# EUROPEAN PATENT SPECIFICATION

(11) **EP 4 539 727 B1**
(45) Date of publication and mention of the grant of the patent: **30.09.2026**
(21) Application number: 23728816.2
(22) Date of filing: 06.06.2023
(51) Int. Cl.: A61B 5/00, A61B 5/02, A61B 5/0215, H04Q 9/04

(54) **METHOD FOR AUTOMATIC BLOOD PRESSURE MEASUREMENT**
VERFAHREN ZUR AUTOMATISCHEN BLUTDRUCKMESSUNG
MÉTHODE DE MESURE AUTOMATIQUE DE LA PRESSION ARTÉRIELLE

(30) Priority: 17.06.2022 EP 22179632
(43) Date of publication of application: 23.04.2025
(73) Proprietor: BIOTRONIK SE & Co. KG, 12359 Berlin (DE)
(72) Inventor: WEGERICH, Franziska, 12055 Berlin (DE); MOSS, Christian, 14612 Falkensee (DE)
(74) Representative: Biotronik Corporate Services SE
(86) International application number: PCT/EP2023/065088
(87) International publication number: WO 2023/241994

(56) References cited:
- US-A1- 2006 122 667
- US-A1- 2016 038 087
- US-A1- 2018 021 585

## Description

The present disclosure generally relates to automatic blood pressure measurement by implantable pressure sensors, and corresponding methods, devices and systems, as well as computer programs.

Implantable pressure sensors have been known for years. They can be implanted, e.g. into a heart and/or a blood vessel to monitor cardiac and/or vascular blood pressure, respectively, at various locations, for example. Also for numerous other applications implantable pressure sensors exist. US 2016/038087 A1 discloses in this regard a stent comprising a pressure sensor. US 2018/021585 A1 discloses leadless implantable medical devices each comprising a pressure sensor. US 2006/122667 A1 discloses a system including implantable medical devices capable of monitoring blood pressure.

In particular, blood pressure sensors have been known for implantation into various positions within a patient. For example, a pressure sensor placed in the pulmonary artery may be used to detect an increased blood pressure in the pulmonary artery early on, which may reduce hospitalization of patients with heart failure. Based on the detected increase, a medication of the patient may be changed and/or introduced, such that events leading to hospitalization may be prevented.

However, many patients suffering from heart failure may suffer from a variety of symptoms, which may still only be detected once the patient him- or herself feels them, i.e. once they exceed a clinical threshold. it would be desirable to detect them earlier, such that exceeding the clinical threshold and the corresponding physical and psychological downsides for the patient may be avoided.

Therefore, there is a need to improve the diagnostic abilities that can be provided by implantable pressure sensors, in particular blood pressure sensors.

This need is at least in part met by the aspects described herein.

According to a first aspect, a method for synchronizing implantable pressure sensors is provided. The method comprises receiving a first inquiry from a first implantable pressure sensor. The method further comprises sending, at a first send time, first measurement time information to the first implantable pressure sensor. The first measurement time information relates to a common measurement time of the first implantable pressure sensor and at least one further implantable pressure sensor.

The above aspect is based on the underlying idea that several pressure sensors, in particular blood pressure sensors, may be implanted at various positions in a patient's body to improve the possibilities of diagnostics. However, in order to do so, it would be highly beneficial to have measurements of the different sensors pertaining to the same instances in time. Since it would not be practical to have the sensors measure at all times (such that measurements relating to the same time but stemming from different sensors would naturally be available), it is beneficial to synchronize the sensors and their measurement times. This is enabled by the above aspect in a particularly beneficial manner. Measurement time information is sent to the first implantable pressure sensor that relates to a common measurement time of the sensor and one or more further sensors. Hence, the first sensor is enabled to measure in synchrony with the further sensors. At the same time, this information is sent upon a corresponding inquiry by the first sensor. Hence, the sensor does not need to be in listen mode at all times to listen for its measurement time. Instead, it sends a corresponding inquiry and only needs to be in listen mode (e.g. having its receiver powered up) shortly thereafter. For example, the inquiry may be sent on a regular basis, e.g. once per day, at which time the sensor may (at least in part) wake up, such that the first sensor may request the current measurement time in an autarkic manner, and such that it may then, later on, follow an orchestrated measurement in synchrony with one or more further sensors. Before that, however, and once the measurement time information has been received by the first sensor, the first sensor may power down to save power, e.g., up until the common measurement time has been reached, at which time (or shortly before that) the first sensor may power up again.

It is noted that the term "implantable" as used herein may refer to a device that is suitable for implantation but has not yet been implanted. It may however also refer to a device that has already been implanted.

Hence, for example a pressure sensor implanted in a pulmonary artery (arteria pulmonalis) of a patient may be operated in synchrony with a pressure sensor implanted in a renal vein (vena renalis) or a vena cava of the same patient. Hence, in addition to the blood pressure in the pulmonary artery (which may be beneficial particularly for monitoring patients with left heart failure and/or problems of pulmonary congestion), also the "right" cardiac blood pressure, e.g., in the renal vein and/or the vena cava (inferior), e.g. central and/or renal venous pressure may be monitored (which may be beneficial for patients with additional right heart failure and/or systemic congestion and/or volume overload). This may for example enable an even more specific adaptation of a certain medication to the needs of each individual patient suffering from left and right congestion symptoms by means of a tailored therapy for cardiac insufficiency. In contrast, if, for patients with left and right cardiac insufficiency, pressure is only measured in the pulmonary artery (for the left side) or only in the vena cava or renal vein (for the right side), this may not allow to provide a clinical picture of the same value for the patient. For example, congestion symptoms may only be detected once they manifest as clinical symptoms discernible for the patient (e.g. a shortness of breath when congestion in the lungs occurs, or an increase in body weight or a swelling of the legs in case of systemic congestion). By the aspects as disclosed herein, these may be detected earlier, and a targeted therapy may thus be started earlier.

Both pressure sensors can in particular automatically determine pressure values at the same time (in programmable periods of time) which is desired for a targeted analysis free from temporal fluctuations of the pressure at various body positions.

In some examples, one or more sensors as described herein may generally be implanted or configured for implanting into the pulmonary artery, the left ventricle, the right ventricle, the left atrium, the right atrium, the vena cava (inferior), and/or the renal vein.

This is enabled by the present disclosure in an efficient, energy-saving manner that allows operation of the correspondingly implanted sensors for a long time without the need for replacement and/or recharging a battery of the sensors.

The first measurement time information indicates a first timer value. Thus, instead of providing the first measurement time information in form of an absolute time, a timer is provided. Hence, deviations and/or drifts over time that may occur in the system times of the various pressure sensors become irrelevant, such that the synchronization is more accurate and more stable over time (or a system time may not be needed at all, such that sensor dimensions may be reduced). The first sensor, and each further sensor taking part in the orchestrated measurement, only needs to count down a timer which is independent from accumulated drifts of system times over time. Moreover, indicating a timer value may also allow transmitting more precise timing information, e.g. assuming the same number of bits used as in an absolute time system, since only a (relatively) short time has to be encoded. Hence, more synchronous measurements may be enabled.

The first timer value is determined at least in part based on the first send time. Hence, potentially different send times may be taken into account, and the send times may be chosen independently from the actual common measurement times. This may allow working with flexible send times and/or measurement times.

The first timer value indicates a remaining time until the common measurement time.

For example, it may indicate a remaining time from the first send time until the common measurement time. For example, the timer value may be determined shortly before the first send time, e.g. taking into account a possible delay from determination of the first timer value until the first send time. In some examples, also a possible delay from sending the first timer value and receiving the first timer value at the first implantable pressure sensor may additionally or alternatively be taken into account. In other, examples, such delay(s) may be neglected, and the timer value may be determined as the remaining time until the common measurement time, when the timer value is determined (this time would then also be considered as first send time, since the delay up until the first send time is neglected).

The method further includes the following steps: receiving a second inquiry from a second implantable pressure sensor; sending, at a second send time, second measurement time information to the second implantable pressure sensor relating to a common measurement time of the first implantable pressure sensor and the second implantable pressure sensor. Hence, two or more implantable pressure sensors are conveniently synchronized to the same common measurement time.

The second measurement time information indicates a second timer value.

The second timer value is determined at least in part based on the second send time.

The second timer value indicates a remaining time until the common measurement time.

The above aspects relating to the second measurement time information and/or second timer value may generally be similar to the aspects outlined with reference to the first measurement time information and/or first timer value. However, the first timer value and the second timer value may differ from each other.

The above aspects allows a particularly flexible synchronization scheme, wherein each implantable pressure sensor may be informed about the common measurement time at a different instant in time, but still, fluctuations in internal absolute timings of the pressure sensors may not matter. Each pressure sensor is in addition enabled to inquire and receive measurement time information essentially at any time, maximizing flexibility and energy-efficiency of the scheme as well as allowing to reduce interference that may particularly be detrimental in (relatively high noise) intrabody communications.

The sending of the first measurement time information may include sending on a frequency portion different from a frequency portion on which the first inquiry was received. For example, the first inquiry may be received on a generic frequency portion that may generally be used by various sensors for communication, in particular for inquiring about the establishing of a communication link. Upon receiving the first inquiry, it may be determined that a different frequency portion is more suitable (e.g. comprises less noise, interference, etc.) for communication, such that the first measurement time information may be transmitted on a different frequency portion, e.g. more reliably.

In some examples, the method may further comprise receiving a reception acknowledgment in response to sending the first measurement time information. This may enable to confirm whether the first implantable pressure sensor has received the first measurement time information and is thus properly synchronized. If an acknowledgement is not received (in a predetermined time window upon sending the first measurement time information), the first measurement time information may be sent again. Hence, it may be confirmed that the first implantable pressure sensor is indeed properly synchronized, essentially on the spot, and not only once the actual measurement data is to be transmitted (e.g. since it is not transmitted at all, or data relating to the wrong period of time is transmitted by the implantable pressure sensor).

According to a further aspect, a method for synchronizing implantable pressure sensors is provided. The method may comprise: transmitting a first inquiry from a first implantable pressure sensor; receiving, at a first receipt time, first measurement time information relating to a common measurement time of the first implantable pressure sensor and at least one further implantable pressure sensor.

The first measurement time information indicates a first timer value. The first timer value indicates a remaining time until the common measurement time. For example, it may indicate a remaining time from the first receipt time until the common measurement time (or a corresponding send time at the transmitter of the first measurement time information; in some examples, these may assumed to be equal in case minor transmission latency is neglected).

The method may further include, for example, prior to transmitting the first inquiry, switching the first implantable pressure sensor into an active communication mode. Additionally or alternatively, it may comprise, after the first receipt time, switching the active communication mode off (e.g. into a passive mode). Further, additionally or alternatively, it may comprise switching the first implantable pressure sensor into an active measurement mode at the common measurement time, and switching the active measurement mode off thereafter (e.g. into a passive mode).

A further aspect relates to a mobile communication apparatus for synchronizing implantable pressure sensors. The mobile communication apparatus may comprise: means for receiving a first inquiry from a first implantable pressure sensor; and means for sending, at a first send time, first measurement time information to the first implantable pressure sensor relating to a common measurement time of the first implantable pressure sensor and at least one further implantable pressure sensor.

The mobile communication apparatus may facilitate implementing the advantageous aspects described herein regarding synchronization of implantable pressure sensors and may in particular be adapted to implement methods outlined herein. The mobile communication apparatus may be implemented as a portable and/or handheld device, such as a tablet, smartphone, etc. or a wearable, such as a smartwatch, etc. The mobile communication apparatus may comprise a transceiver for receiving and/or transmitting signals to an implantable pressure sensor. For example, a wireless communication method may be used for that matter, e.g. Medical Implant Communication Service (MICS), Bluetooth (Low Energy), and/or Near Field Communication (NFC). The mobile communication apparatus may communicate with the implantable pressure sensor(s) directly, and/or via a relay device, e.g. an implantable relay device.

A yet further aspect relates to an implantable pressure sensor for synchronizing with at least one further implantable pressure sensor. The implantable pressure sensor may comprise: means for transmitting a first inquiry from the first implantable pressure sensor;
means for receiving, at a first receipt time, first measurement time information relating to a common measurement time of the first implantable pressure sensor and the at least one further implantable pressure sensor.

The implantable pressure sensor may comprise a transceiver similarly as outlined with reference to the mobile communication apparatus, for example. In some examples, the means for transmitting may be configured to transmit the first inquiry at a first predetermined time. The transmitting may be configured, e.g. programmed, to occur regularly, e.g. at a first predetermined time, every day (e.g. at 6 am, for example).

A further aspect relates to a system comprising at least one implantable pressure sensor and a mobile communication apparatus as described herein.

Yet another aspect relates to a method that may be carried out by such a system and that comprises method steps as outlined herein with reference to the mobile communication apparatus and the implantable pressure sensor(s).

Finally, a further aspect relates to a computer program that comprises instructions which, when carried out by a processor cause the processor to carry out the steps according to a method as described herein. The computer program may, for example be stored on a storage medium of a mobile communication apparatus and/or an implantable pressure sensor.

It is noted that the method steps as described herein may include all aspects described herein, even if not expressly described as method steps but rather with reference to an apparatus (or device). Moreover, the apparatuses as outlined herein may include means for implementing all aspects as outlined herein, even if these may rather be described in the context of method steps.

Whether described as method steps, computer program and/or means, the functions described herein may be implemented in hardware, software, firmware, and/or combinations thereof. If implemented in software/firmware, the functions may be stored on or transmitted as one or more instructions or code on a computer-readable medium. Computer-readable media include both computer storage media and communication media including any medium that facilitates transfer of a computer program from one place to another. A storage medium may be any available media that can be accessed by a general purpose or special purpose computer. By way of example, and not limitation, such computer-readable storage media can comprise RAM, ROM, EEPROM, FPGA, CD/DVD or other optical disk storage, magnetic disk storage or other magnetic storage devices, or any other medium that can be used to carry or store desired program code means in the form of instructions or data structures and that can be accessed by a general-purpose or special-purpose computer, or a general-purpose or special-purpose processor. The control unit as described herein may also be implemented in hardware, software, firmware, and/or combinations thereof, for example, by means of one or more general-purpose or special-purpose computers, and/or a general-purpose or special-purpose processors.

Aspects of the present disclosure are described in more detail in the following by reference to the accompanying Figures. The Figures show:
- Fig. 1: an exemplary arrangement of two implanted pressure sensors;
- Fig. 2: an exemplary schematic of two implanted pressure sensors in communication with a mobile communication apparatus;
- Fig. 3: an exemplary communication protocol between a first implantable pressure sensor and a mobile communication apparatus;
- Fig. 4: an exemplary communication protocol between a second implantable pressure sensor and a mobile communication apparatus.

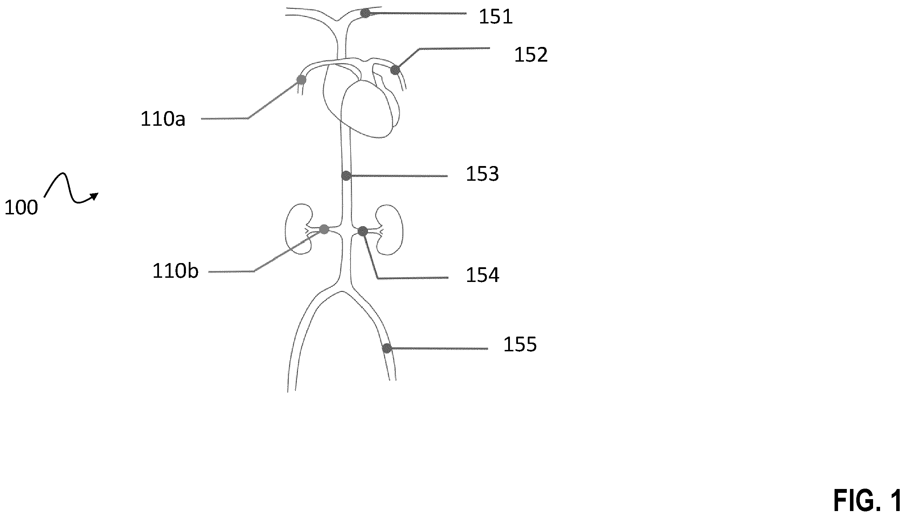

In the following, exemplary embodiments of the present disclosure are described in more detail, primarily with reference to Figs. 1-4. While specific feature combinations are described in the following with respect to the exemplary embodiments, it is to be understood that the disclosure is not limited to such embodiments. In particular, various further embodiments may be realized with less and/or different features as expressly described in the following, as will be readily understood by the skilled person.

Fig. 1 shows an exemplary arrangement 100 of two implantable pressure sensors 110a and 110b implanted into exemplary locations within a patient's body.

First pressure sensor 110a is implanted into an arteria pulmonalis 152 of the patient. First pressure sensor 110a may be implanted into the left and/or right arteria pulmonalis 152. It may be placed at this position, e.g. after having been introduced via vena subclavia 151 or vena femoralis 155. First pressure sensor 110a may generally comprise a pressure transducer that may be powered by a (wirelessly rechargeable) battery. It may comprise an AD converter for digitizing signals received from the pressure transducer, and a processing unit (such as a processor, microcontroller, ASIC, etc.) that may process the digitized signals, for example. Further, it may comprise a transceiver for performing the sending and receiving as described herein. A further processing unit (such as a processor, microcontroller, ASIC, etc.) and/or the already mentioned processing unit may control operation of first pressure sensor 110a, e.g. it may trigger the sending of an inquiry (e.g. at a predetermined, e.g., programmed time and/or after the lapse of a predetermined, e.g. programmed, timer) and/or the sending of measurement data (e.g. at a predetermined, e.g., programmed time and/or after the lapse of a predetermined, e.g. programmed, timer), etc. Further, pressure sensor 110a may comprise one or more fixation elements, such as wires and/or loops etc. for fixation to tissue of the patient, e.g. the wall of a blood vessel and/or the heart. The fixation elements may be adapted to the specifically intended site of implantation, e.g. the size of the arteria pulmonalis 152, in the example of Fig. 1.

Second pressure sensor 110b may be essentially similar to first pressure sensor 110a. However, it may be implanted in a vena renalis 154 (for example the left or the right vena renalis). Consequently, its fixation element(s), specifically their size, may be adapted accordingly. Second pressure sensor 110b may be inserted via vena femoralis 155, for example.

It is noted that in other embodiments, second pressure sensor 110b may be implanted into the vena cava 153 (specifically for example the vena cava inferior). Its fixation element(s) may be adapted in size accordingly. It may also be inserted via vena femoralis 155.

The arrangement of first and second pressure sensors 110a and 110b in the arteria pulmonalis 152 and the vena renalis 154 and/or vena renalis 154 may allow simultaneous pressure measurement at two relevant positions. At both positions, (measurably) increased pressure values may arise with patients having heart failure before clinical symptoms occur.

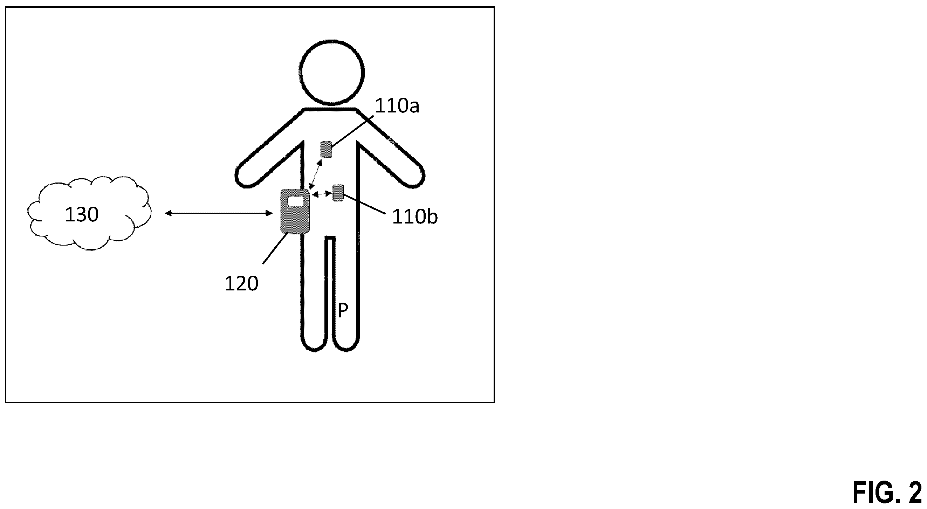

Fig. 2 shows an exemplary schematic of pressure sensors 110a and 110b implanted into patient P and communicating with mobile communication apparatus 120. It is noted that only two pressure sensors are included in this embodiment for easier illustration. However, it is understood that, e.g. three, four, etc. or generally a plurality of pressor sensors may be included.

Mobile communication apparatus 120 may be implemented as a patient device (e.g. a device intended to be carried and/or worn by patient P and/or a device intended to be positioned next to the patient's bed, for example). Mobile communication apparatus 120 may be in communication with remote communication system 130. To this end, mobile communication apparatus may comprise a suitable wireless interface, such as WiFi, 3G, 4G and/or 5G, for example. Communication with remote communication system 130 may be via a mobile radio communications system or the internet, for example.

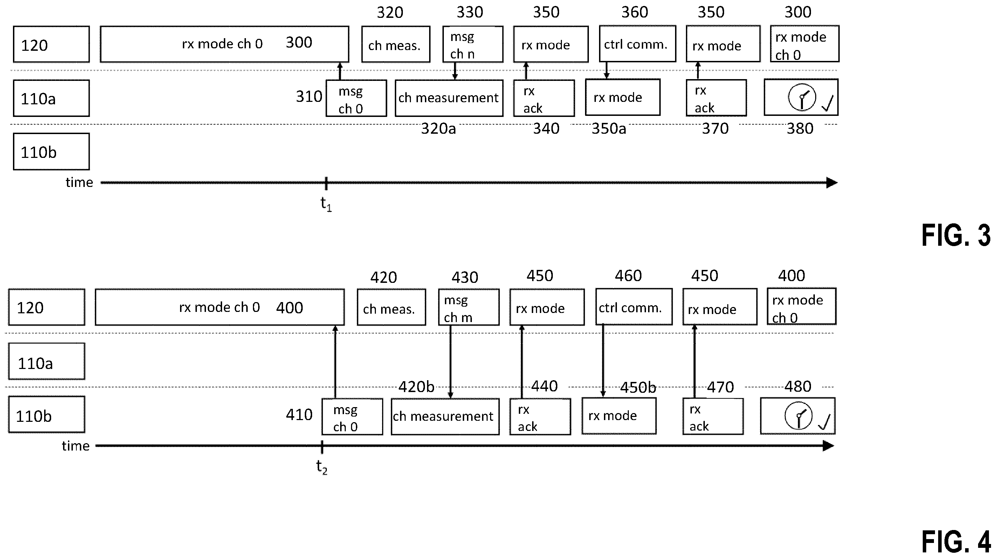

Mobile communication apparatus 120 may facilitate synchronization of pressure sensors 110a and 110b with each other, e.g., according to a protocol as outlined with reference to Figs. 3 and 4 that includes first and second timer values.

First and second pressure sensors 110a and 110b may generally be in a passive (sleep) mode. At the common measurement time, they may wake up, i.e. the elements of each pressure sensor necessary for pressure measurement (e.g. processing unit and pressure transducer) may be powered up. The powering up may be triggered by a clock, oscillator or any similar timing means of each pressure sensor that counts down a timer and may, e.g., be in communication with the processing unit (the latter may be powered up by the trigger and/or the latter may power up e.g. the pressure transducer, and possibly also the transceiver, as the case may be). Each pressure sensor may then acquire measurement data pertaining to the blood pressure at the sites of implantation, respectively.

First and second pressure sensors 100a and 100b may then transmit pressure data acquired at the common measurement time to mobile communication apparatus 120. The transmission may occur, e.g. directly after the measurement data has been acquired by the respective pressure sensors 110a, 100b, and after the corresponding transmitter has been powered up, immediately before transmission occurs, at the latest (e.g. if it was not powered up already together with the pressure transducer). The powering up may be triggered by a clock, oscillator or any similar timing means of each pressure sensor that counts down a timer and may, e.g., be in communication with the processing unit.

It may be conceivable that the elements necessary for pressure measurement are then powered down, together with the transmitter after transmission. Alternatively, the elements necessary for pressure measurement may already be powered down earlier, e.g. after the measurement data has been acquired.

It is also possible that the pressure data is sent by the first and second pressure sensors 100a and 100b at different times, for example, to avoid interference. For example, at least one of the sensors may send the pressure data, e.g. at a predetermined time after the measurement data has been acquired. For example, the measurement data of the first pressure sensor 100a may be sent after a first predetermined sending timer lapses at the first pressure sensor 100a, and/or the measurement data of the second pressure sensor 100b may be sent after a second predetermined sending timer (different from the first predetermined sending timer) lapses at the second pressure sensor 100b. Also in this case, the transmitter may be powered down after transmission has been accomplished by the respective pressure sensor.

In other examples, transmitting the measurement data may include sending an inquiry, as will be outlined further below.

First and/or second pressure sensor 100a/100b may then wake up once again, e.g., to transmit an inquiry to mobile communication apparatus 120 (to request the next measurement time information). This may be done at a predetermined time and/or after a corresponding predetermined first and second send timer lapses, respectively (that may e.g. be initialized upon sending the previous pressure data). It is also conceivable that the inquiry is sent, by each respective pressure sensor, directly after the (previous) pressure data has been transmitted. Thus, a separate wake-up of the transmitter (for sending the inquiry and receiving the next measurement time information) may be avoided.

Mobile communication apparatus 120 may monitor and/or process the pressure data received from pressure sensors 100a/100b. Additionally or alternatively, it may forward the pressure data, possibly together with further data, to remote communication system 200. Remote communication system may be a cloud and/or server based monitoring system, for example, that allows access to medical staff and/or hospitals in order to monitor patients. It may be configured to issue a warning and/or an alarm to medical staff and/or the patient in case abnormal behavior is determined based on the pressure data supplied by the first and second pressure sensors 100a, 100b.

Fig. 3 shows an exemplary protocol for synchronizing first pressure sensor 110a by means of mobile communication apparatus 120 such that first pressure sensor 110a can measure at a common measurement time with second pressure sensor 110b. The protocol will be outlined within a framework of MICS. However, it is noted that similar protocols may also be implemented with other communication services and/or systems.

Initially, mobile communication apparatus 120 is generally in a receive mode 300, wherein it monitors a common receive channel. Common receive channel may be characterized by a specific frequency bandwidth known to and used by implantable pressure sensors 110a and 110b to send inquiries. In MICS, it may be referred to as channel 0 and may be located at a frequency of 403.65 MHz, for example.

At a first predetermined time t₁, first implantable pressure sensor 110a may send a message 310 that includes an inquiry to mobile communication apparatus 120 on the common receive channel. The inquiry may relate to a request for measurement time information (in other embodiments it may relate to a request for a data transmission channel). The first predetermined time may be based on a time and/or a timer programmed into implantable pressure sensor 110a, e.g. as outlined with reference to Fig. 2. For example, a message 310 may be sent regularly, e.g. at least once a day.

After having sent message 310, first pressure sensor 110a may transition into a channel measurement mode 320a, wherein it is listening to various channels (e.g. in the MICS band) for a response from mobile communication apparatus 120. This mode may be characterized in that the first pressure sensor 110a is able to recognize the channel with the highest signal amplitude (which is then highly likely that on which the response has been received, particularly due to the close proximity of mobile communication apparatus 120 and first implantable pressure sensor 110a).

Upon reception of message 310, mobile communication apparatus 120 transitions into a channel measurement mode 320. Therein, it may measure suitable channels for communication with first implantable pressure sensor 110a and/or select a suitable channel. In particular, it may determine whether one or more channels (e.g. in the MICS frequency band) is already occupied for communication with one or more other implants.

Upon selection of a channel (ch n), mobile communication apparatus 120 transmits a message 330 on the selected channel to first pressure sensor 110a which allows first pressure sensor 110a to recognize the selected channel. Mobile communication apparatus 120 may then transition into a receive mode 350 in which it monitors the selected channel.

Upon reception of message 330 on the selected channel by first pressure sensor 110a, the latter may send a receive acknowledgement 340 on the selected channel. Subsequently, first pressure sensor 110a may then transition into a receive mode 350a in which it monitors the selected channel.

Upon reception of acknowledgement 340, mobile communication apparatus 120 may send a control communication 360 to first pressure sensor 110a. Control communication 360 may comprise measurement time information as described herein. Additionally, it may comprise further information, e.g. for programming first pressure sensor 110a and/or information on whether first pressure sensor 110a is the first, second, etc. pressure sensor in the synchronization sequence. The first measurement time information and/or the further information may be relayed by mobile communication apparatus 120 as received from a remote communication system 130 (not shown in Fig. 3), for example.

First pressure sensor 110a may respond to the received control communication 360 by a corresponding receive acknowledgement 370. It may then set the measurement time 380 to the received common measurement time information, e.g. it may start a timer, as described herein. It may then power off, until it wakes up a the common measurement time 380.

Mobile communication apparatus 120 may, upon reception of receive acknowledgment 370, fall back into receive mode 300, wherein it monitors a common receive channel.

It is noted that message 310 may be sent two, three or in general a predetermined number of times if no (response) message from mobile communication apparatus 120 is detected by first implantable pressure sensor 110a, after a predetermined time. After that, the sequence may be aborted, as it may be assumed that mobile communication apparatus 120 is presently unavailable. Similarly, mobile communication apparatus 120 may send message 330 and control communication 360 two, three or in general a predetermined number of times if no corresponding acknowledgement is received from first implantable pressure sensor 110a, after a predetermined time. After that, the sequence may be aborted, as it may be assumed that first pressure sensor 110a is presently unavailable. In that case, also an alarm may be issued and/or sent to remote communication system 130.

The described search for a free transmission channel (listen before talk) is particularly adapted to conditions of miniaturized implants with small antennas and low power transmission.

Fig. 4 shows an exemplary protocol for synchronizing second pressure sensor 110b by means of mobile communication apparatus 120 such that second pressure sensor 110b can measure at a common measurement time with first pressure sensor 110a. It is generally similar to the protocol outlined with reference to Fig. 3, with the roles of first and second pressure sensors 110a, 110b interchanged.

Generally, mobile communication apparatus 120 may initially be in a receive mode 400, which may be similar to receive mode 300.

At a second predetermined time t₂, second implantable pressure sensor 110b may send a message 410 that includes an inquiry to mobile communication apparatus 120 on the common receive channel. The inquiry may relate to a request for measurement time information (in other embodiments it may relate to a request for a data transmission channel). The second predetermined time may be based on a time and/or a timer programmed into implantable pressure sensor 110b, e.g. as outlined with reference to Figs. 2 and 3. Particularly, second predetermined time t₂ may differ from first predetermined time t₁. For example, each pressure sensor (designated for implantation to the same patient) may comprise its unique predetermined time for sending the inquiry.

After having sent message 410, second pressure sensor 110b may transition into a channel measurement mode 420b, wherein it is listening to various channels (e.g. in the MICS band) for a response from mobile communication apparatus 120. This mode may generally be similar to mode 320a outlined with reference to first pressure sensor 110a in Fig. 3.

Upon reception of message 410, mobile communication apparatus 120 transitions into a channel measurement mode 420, similarly as outlined with reference to mode 320 in Fig. 3. Therein, it may measure suitable channels for communication with second implantable pressure sensor 100b and/or select a suitable channel. In particular, it may determine whether one or more channels (e.g. in the MICS frequency band) is/are already occupied for communication with one or more other implants.

Upon selection of a channel (ch m, which may or may not be identical to ch n), mobile communication apparatus 120 transmits a message 430 on the selected channel to second pressure sensor 110b which allows second pressure sensor 110b to recognize the selected channel. Mobile communication apparatus 120 may then transition into a receive mode 450 in which it monitors the selected channel.

Upon reception of message 430 on the selected channel by second pressure sensor 110b, the latter may send a receive acknowledgement 440 on the selected channel. Subsequently, second pressure sensor 110b may then transition into a receive mode 450b in which it monitors the selected channel.

Upon reception of acknowledgement 440, mobile communication apparatus 120 may send a control communication 460 to second pressure sensor 110b. Control communication 460 may comprise measurement time information as described herein. Additionally, it may comprise further information, e.g. for programming second pressure sensor 110b and/or informing the second pressure sensor 100b that it is second, third, fourth, ..., in the sequence. The first measurement time information and/or the further information may be relayed by mobile communication apparatus 120 as received from a remote communication system 130 (not shown in Fig. 3), for example.

Second pressure sensor 110b may respond to the received control communication 460 by a corresponding receive acknowledgement 470. It may then set the measurement time 480 to the received common measurement time information, e.g. it may start a timer, as described herein. It may then power off, until it wakes up at the common measurement time 480.

Mobile communication apparatus 120 may, upon reception of receive acknowledgment 470, fall back into receive mode 400, wherein it monitors a common receive channel.

It is noted that message 410, message 430 and/or control communication 460 may be sent two, three or in general a predetermined number of times similarly as outlined with reference to message 310, message 330 and control communication 360 of Fig. 3.

Further, it is noted that transmission of measurement data from first implantable pressure sensor 110a and/or second implantable pressure sensor 110 may occur according to a similar protocol as that outlined with reference to Figs. 3 and 4, respectively.

For example, at the time the first pressure sensor 110a intends to transmit its measurement data, it may transmit a message with an inquiry for data transmission which may generally be similar to message 310 outlined with reference to Fig. 3 (while mobile communication apparatus 120 is in a receive mode similar to receive mode 300 described with reference to Fig. 3). That time may be predetermined, e.g., as outlined herein (e.g. with reference to time t₁). First pressure sensor 110a may then transition into a channel measurement mode similar to mode 320a. Mobile communication apparatus 120 may perform a channel measurement and selection, similarly as described with reference to mode 320. It may then send a message to first pressure sensor 110a indicating the selected channel, which may be similar to message 330, and then switch to a receive mode in which the selected channel is monitored (similarly as in mode 350). The first pressure sensor may, upon reception of the message, reply with a receive acknowledgement (similar to acknowledgement 340), and also switch to a receive mode in which it monitors the selected channel (similarly as in mode 350a).

Then, first pressure sensor 110a may send the measurement data (e.g. of the preceding common measurement time) to mobile communication apparatus 120. Mobile communication apparatus may respond with an acknowledgement and then re-enter the mentioned receive mode for the common channel similar to receive mode 300 described with reference to Fig. 3. First pressure sensor 110a may power-down (e.g. passive mode) upon reception of the acknowledgement. If no acknowledgement has been received after a predetermined time (after transmission), it may resend the data, e.g. until a maximum number of retransmissions has been reached.

The transmission of measurement data by the second implantable pressure sensor 110b may proceed similarly. However, it may send the inquiry to transmit measurement data at a time different from that used by the first implantable pressure sensor 110a. Similar considerations apply with respect to these times as those outlined with reference to times t₁ and t₂ of Figs. 3 and 4, respectively. In particular, each pressure sensor (designated for implantation to the same patient) may comprise its unique predetermined time for that matter.

In some examples, the first pressure sensor 110a, that may be informed that it is the first in the sequence, may send the inquiry for data transmission directly after having acquired the measurement data. In particular, the inquiry may be sent several times, e.g. similarly as outlined with reference to message 310 of Fig. 3. Upon successful connection establishment, the pressure data may be transmitted. The second pressure sensor 110b, that may be informed that it is the second (or third, ...) in the sequence may, in that example, send its inquiry for data transmission, e.g. after a timer (that may generally be programmed to correspond to second (or third, ...) devices) after completion of the measurement has lapsed. The timer (corresponding to second, third, etc. devices) may be programmed into an implantable device at manufacture, for example, such that regardless of whether it will be used as first, second, etc. sensor, the corresponding timer will be known to the sensor. Additionally or alternatively, the timing may be determined during use, e.g., by means of programming via the mobile communication apparatus 120.

Once mobile communication apparatus 120 has acquired measurement data pertaining to the common measurement time from all relevant sensors, it may transmit the data, possibly together with further data, to remote communication system 130, e.g., via a mobile communication network (e.g. 3G, 4G, 5G), e.g. for processing of the data at remote communication system 130. The data may be processed before transmission, e.g. the data from the relevant sensors may be transformed into a joint data-set for the patient.

In summary, the measurement times and/or intervals of various implantable pressure sensors 110a, 110b may be controlled e.g. by the mobile communication apparatus 120 and/or by remote communication system 130 (e.g. via mobile communication apparatus 120). Also, in the same manner, measurement times and/or intervals of the sensors may be synchronized. The aspects described herein thus enable automatic pressure measurements in an energy efficient manner such that the sensors may operate for a long time (without having to recharge batteries, for example).

An important aspect is that the communication may always start from a respective implantable pressure sensor 110a, 110b, and not from mobile communication apparatus 120. This renders it unnecessary that the implantable pressure sensors 110a, 110b need to monitor received signals at all times or at least intermittently. Instead, this task is outsourced to mobile communication apparatus 120 which may allow much easier recharging and larger batteries.

## Claims

1. A method for synchronizing implantable blood pressure sensors (110a, 110b), the method comprising:
receiving a first inquiry (310) from a first implantable blood pressure sensor (110a);
sending, at a first send time and upon receiving the first inquiry, first measurement time information (360) to the first implantable blood pressure sensor (110a) relating to a common measurement time of the first implantable blood pressure sensor (110a) and at least one further implantable blood pressure sensor (110b), wherein the first measurement time information (360) indicates a first timer value which is at least in part based on the first send time, the method further comprising:
receiving a second inquiry (410) from a second implantable blood pressure sensor (110b);
sending, at a second send time and upon receiving the second inquiry, second measurement time information (460) to the second implantable blood pressure sensor (110b) relating to a common measurement time of the first implantable blood pressure sensor (110a) and the second implantable blood pressure sensor (110b), wherein the second measurement time information (460) indicates a second timer value,
**characterized in that** the first timer value indicates a remaining time until the common measurement time, and the second timer value indicates a remaining time until the common measurement time, wherein the first timer value and the second timer value differ from each other.

2. The method according to claim 1, wherein the first pressure sensor (110a) recognizes a channel with the highest signal amplitude.

3. The method according to claim 1 or 2, wherein the sending of the first measurement time information (360) includes sending on a frequency portion different from a frequency portion on which the first inquiry was received.

4. The method according to any of claims 1-3, further comprising receiving a reception acknowledgment (370) in response to sending the first measurement time information (360).

## Patentansprüche

1. Verfahren zum Synchronisieren implantierbarer Blutdrucksensoren (110a, 110b), wobei das Verfahren Folgendes umfasst:
Empfangen einer ersten Anfrage (310) von einem ersten implantierbaren Blutdrucksensor (110a);
Senden, zu einer ersten Sendezeit und beim Empfangen der ersten Anfrage, von ersten Messzeitinformationen (360) zu dem ersten implantierbaren Blutdrucksensor (110a) in Bezug auf eine gemeinsame Messzeit des ersten implantierbaren Blutdrucksensors (110a) und mindestens eines weiteren implantierbaren Blutdrucksensors (110b), wobei die ersten Messzeitinformationen (360) einen ersten Zeitgeberwert angeben, der zumindest teilweise auf der ersten Sendezeit basiert, wobei das Verfahren ferner Folgendes umfasst:
Empfangen einer zweiten Anfrage (410) von einem zweiten implantierbaren Blutdrucksensor (110b);
Senden, zu einer zweiten Sendezeit und beim Empfangen der zweiten Anfrage, von zweiten Messzeitinformationen (460) zu dem zweiten implantierbaren Blutdrucksensor (110b) in Bezug auf eine gemeinsame Messzeit des ersten implantierbaren Blutdrucksensors (110a) und des zweiten implantierbaren Blutdrucksensors (110b), wobei die zweiten Messzeitinformationen (460) einen zweiten Zeitgeberwert angeben,
**dadurch gekennzeichnet, dass** der erste Zeitgeberwert eine verbleibende Zeit bis zur gemeinsamen Messzeit angibt und der zweite Zeitgeberwert eine verbleibende Zeit bis zur gemeinsamen Messzeit angibt, wobei sich der erste Zeitgeberwert und der zweite Zeitgeberwert voneinander unterscheiden.

2. Verfahren nach Anspruch 1, wobei der erste Drucksensor (110a) einen Kanal mit der höchsten Signalamplitude erkennt.

3. Verfahren nach Anspruch 1 oder 2, wobei das Senden der ersten Messzeitinformationen (360) Senden auf einem Frequenzabschnitt beinhaltet, der sich von einem Frequenzabschnitt unterscheidet, auf dem die erste Anfrage empfangen wurde.

4. Verfahren nach einem der Ansprüche 1-3, ferner umfassend Empfangen einer Empfangsbestätigung (370) als Reaktion auf das Senden der ersten Messzeitinformationen (360).

## Revendications

1. Procédé de synchronisation de capteurs de pression artérielle implantables (110a, 110b), le procédé comprenant les étapes consistant à :
recevoir une première interrogation (310) depuis un premier capteur de pression artérielle implantable (110a) ;
envoyer, à un premier temps d'envoi et lors de la réception de la première interrogation, des premières informations de temps de mesure (360) au premier capteur de pression artérielle implantable (110a) relatives à un temps de mesure commun du premier capteur de pression artérielle implantable (110a) et d'au moins un capteur de pression artérielle implantable (110b) supplémentaire, dans lequel les premières informations de temps de mesure (360) indiquent une première valeur de chronomètre qui est au moins en partie basée sur le premier temps d'envoi, le procédé comprenant en outre les étapes consistant à :
recevoir une deuxième interrogation (410) depuis un deuxième capteur de pression artérielle implantable (110b) ;
envoyer, à un deuxième temps d'envoi et lors de la réception de la deuxième interrogation, des deuxièmes informations de temps de mesure (460) au deuxième capteur de pression artérielle implantable (110b) relatives à un temps de mesure commun du premier capteur de pression artérielle implantable (110a) et du deuxième capteur de pression artérielle implantable (110b), dans lequel les deuxièmes informations de temps de mesure (460) indiquent une deuxième valeur de chronomètre,
**caractérisé en ce que** la première valeur de chronomètre indique un temps restant avant le temps de mesure commun, et la deuxième valeur de chronomètre indique un temps restant avant le temps de mesure commun, dans lequel la première valeur de chronomètre et la deuxième valeur de chronomètre différent l'une de l'autre.

2. Procédé selon la revendication 1, dans lequel le premier capteur de pression (110a) reconnaît un canal avec l'amplitude de signal la plus élevée.

3. Procédé selon la revendication 1 ou 2, dans lequel l'étape consistant à envoyer les premières informations de temps de mesure (360) inclut une étape consistant à envoyer sur une portion de fréquence différente d'une portion de fréquence sur laquelle la première interrogation a été reçue.

4. Procédé selon l'une quelconque des revendications 1 à 3, comprenant en outre une étape consistant à recevoir une confirmation de réception (370) en réponse à l'étape consistant à envoyer les premières informations de temps de mesure (360).
